# EUROPÄISCHE PATENTANMELDUNG

(11) **EP 3 420 990 A1**
(43) Veröffentlichungstag der Anmeldung: **02.01.2019**
(21) Anmeldenummer: 18176754.2
(22) Anmeldetag: 08.06.2018
(51) Int. Cl.: A61B 17/70, A61B 90/00

(54) **VERLÄNGERUNGSVORRICHTUNG FÜR EINEN KNOCHENANKER**

(30) Priorität: 27.06.2017 DE 102017114267
(71) Anmelder: Silony Medical International AG, 8500 Frauenfeld (CH)
(72) Erfinder: HEUER, Frank, 70794 Filderstadt (DE)
(74) Vertreter: DREISS Patentanwälte PartG mbB

(57) **Zusammenfassung**

Vorliegende Erfindung betrifft eine Verlängerungsvorrichtung (2) für einen Knochenanker (4), insbesondere für eine Knochenschraube, insbesondere für eine Pedikelschraube, insbesondere in der minimalinvasiven Wirbelsäulenchirurgie, mit einer axialen Längsrichtung (16), einer hierzu konzentrischen Umfangsrichtung (18) und einer radialen Richtung (20), wobei die Verlängerungsvorrichtung (2) in der axialen Längsrichtung (16) erstreckt ist und mittels eines radialen Vorsprungs (60) einen hintergreifbaren Bereich (14) am Kopf (8) des Knochenankers (4) in der Längsrichtung (16) und zudem in radialer Richtung 20 hintergreift und in entgegengesetzter Richtung gegen den Kopf (8) des Knochenankers (4) abstützbar ist, so dass die Verlängerungsvorrichtung hierdurch am Kopf (8) des Knochenankers lösbar, jedoch starr und zudem drehfest fixierbar ist.

## Beschreibung

Die Erfindung betrifft eine Verlängerungsvorrichtung für einen Knochenanker, insbesondere für eine Knochenschraube, insbesondere für eine Pedikelschraube, insbesondere in der minimalinvasiven Wirbelsäulenchirurgie, mit einer axialen Längsrichtung, einer hierzu konzentrischen Umfangsrichtung und einer radialen Richtung, wobei die Verlängerungsvorrichtung in der axialen Längsrichtung erstreckt ist und mittels eines radialen Vorsprungs einen hintergreifbaren Bereich am Kopf des Knochenankers in der Längsrichtung und zudem in radialer Richtung hintergreift und in entgegengesetzter Richtung gegen den Kopf des Knochenankers abstützbar ist, so dass die Verlängerungsvorrichtung hierdurch am Kopf des Knochenankers lösbar, jedoch in der Längsrichtung starr und zudem drehfest fixierbar ist.

Verlängerungsvorrichtungen der vorstehend genannten Art finden bevorzugt bei minimalinvasiven Eingriffen, insbesondere zur Implantation von Knochenankern Verwendung. Sie sollen während des chirurgischen Eingriffs gewissermaßen einen Arbeitskanal zu dem Knochenanker zugänglich halten, durch den der Chirurg mittels weiterer Instrumente den Knochenanker befestigen und gegebenenfalls weitere Implantatteile zuführen und befestigen kann und repositionierende Maßnahmen ausführen kann.

Eine Verlängerungsvorrichtung mit zwei voneinander separaten Schalenteilen ist beispielsweise bekannt aus WO 2007/021588 A1. Diese Schalenteile müssen mittels eines weiteren beide Teile verbindenden Montageteils am Kopf des Knochenankers befestigt werden. Ein radialer Hintergriff der Schalenteile am Kopf des Knochenankers scheint nicht vorgesehen zu sein.

Eine Verlängerungsvorrichtung mit zwei Schalenteilen, die aber proximal einstückig ineinander übergehen, ist bekannt aus US 2016/0183933A1. Die Schalenteile weisen jeweils ein Schwenkhebelelement zur Ausbildung eines Hintergriffs mit dem Knochenanker auf.

Der vorliegenden Erfindung liegt die Aufgabe zugrunde, eine Verlängerungsvorrichtung für einen Knochenanker zu schaffen, die auf einfache Weise vor oder nach der Implantation des Knochenankers am Kopf des Knochenankers lösbar festlegbar ist, wobei ein unbeabsichtigtes Ablösen der Verlängerungsvorrichtung sicher vermieden werden können soll.

Diese Aufgabe wird durch eine Verlängerungsvorrichtung mit den Merkmalen des Anspruchs 1 gelöst.

Obschon vorzugsweise zwei Schalenteile zum Einsatz gelangen, damit diese beiden Schalenteile einen Arbeitskanal bilden können, ist es grundsätzlich denkbar, in bestimmten Situationen mit nur einem Schalenteil zu arbeiten. Werden zwei Schalenteile eingesetzt, so erweist es sich als vorteilhaft, wenn sie beide entsprechend der Erfindung und vorzugsweise identisch bzw. einander entsprechend ausgebildet sind.

Durch die erfindungsgemäße Verwendung des federelastischen oder mit einem federelastischen Abschnitt ausgebildeten Schwenkhebelelements der beanspruchten Art lässt sich unter Verwendung desselben Elements eine Umlenkung einer bei der Montage des Schalenteils am Kopf des Knochenankers ausgeübten Kraft erzielen, welche dann in einer anderen Richtung bzw. bezüglich eines anderen Freiheitsgrads eine Stellbewegung und/oder Fixierung und/oder Kraftausübung bewirkt. Durch die Ausgestaltung des Schwenkhebelelements, insbesondere durch die geometrische Gestaltung von Hebelarmen des Schwenkhebelelements, und durch die Gestaltung und Anordnung des im Wesentlichen starren Abschnitts und des federelastischen Abschnitts lässt sich auch eine mechanischen Gesetzen folgende Kraftverstärkung durch Einsatz der Hebelwirkung erzielen. - Wenn vorliegend von einem Schwenkhebelelement die Rede ist, so bedeutet dies nicht zwingend, dass dieses Schwenkhebelelement einen diskreten Lagerpunkt mit einer starren Lagerachse aufweisen muss. Das Schwenkhebelelement könnte auch in einem Lagerbereich gewissermaßen schwimmend verschwenkbar aufgenommen sein.

Es kann sich als vorteilhaft erweisen, wenn das Schwenkhebelelement mit seiner zweiten Kontaktzone gegen ein proximales Ende des Kopfs des Knochenankers anlegbar ist. Solchenfalls ist es nicht erforderlich, seitlich am Kopf eine axiale Gegenlagerstelle zu schaffen.

Es kann sich als vorteilhaft erweisen, wenn der federelastische Abschnitt des Schwenkhebelelements im Bereich der ersten Kontaktzone und/oder im Bereich der zweiten Kontaktzone ausgebildet ist.

Es kann sich auch als vorteilhaft erweisen, wenn der im Wesentlichen starre Abschnitt des Schwenkhebelelements zwischen der ersten Kontaktzone und der zweiten Kontaktzone ausgebildet ist. Dies eröffnet die Möglichkeit, dass der im Wesentlichen starre Abschnitt in einem Schwenklagerbereich des Schwenkhebelelements ausgebildet ist und dort für eine unmittelbare, also nicht elastisch absorbierende Kraftweiterleitung, sorgt.

Es kann sich auch als vorteilhaft erweisen, wenn der im Wesentlichen starre Abschnitt des Schwenkhebelelements im Bereich der zweiten Kontaktzone ausgebildet ist. In diesem Fall kann sichergestellt werden, dass die Stellkräfte im Bereich der zweiten Kontaktzone unvermindert, also ohne dort weiter elastisch absorbiert zu werden, auf den Knochenanker eingeleitet werden können.

Es erweist sich weiter als vorteilhaft, wenn das Schwenkhebelelement in einer Ebene erstreckt ist, die insbesondere parallel zur Längsrichtung erstreckt ist. Dies eröffnet die Möglichkeit, dass das Schwenkhebelelement als ebenes Bauteil insbesondere mit gleichförmiger Dicke hergestellt werden kann. Es kann solchenfalls aus einem Flachmaterialabschnitt hergestellt werden, oder es lässt sich auf einfache Weise in anderer Form, etwa als Spritzgussteil oder in einem additiven Herstellungsverfahren, prozesssicher und wirtschaftlich herstellen. Wenn das Schwenkhebelelement als ebenes Bauteil ausgebildet ist, so ist auch seine Anbringung und Lagerung bei der Verlängerungsvorrichtung weniger komplex.

Bei einer Ausführungsform der Erfindung kann das Schwenkhebelelement ungefähr L-förmig ausgebildet sein.

Bei einer anderen Ausführungsform der Erfindung ist das Schwenkhebelelement ungefähr U-förmig ausgebildet und weist zwei Schenkel auf und die zwei Schenkel weisen jeweils eine erste Kontaktzone auf. Durch eine U-förmige Ausbildung des Schwenkhebelelements lässt sich eine Kombination von zwei Schwenkhebelelementen in einem Bauteil erzielen, was sich herstellungstechnisch als vorteilhaft und wirtschaftlich erweist. Dabei ist denkbar, dass das U-förmige Schwenkhebelelement nicht zwingend in einer Ebene erstreckt ist, sondern es kann eine Krümmung, insbesondere im Bereich eines die beiden Schenkel verbindenden Querstegs aufweisen.

Weiter wird vorgeschlagen, dass das Schwenkhebelelement eine Schwenkebene aufweist, die parallel zu der Längsrichtung jedoch nach radial außen versetzt zu dieser verläuft. Ferner wird vorgeschlagen, dass das Schwenkhebelelement eine Schwenk-oder Lagerachse aufweist, die orthogonal zu der Längsrichtung jedoch nach radial außen versetzt zu dieser verläuft. Durch die Versetzung des Schwenkhebelelements nach radial außen lässt sich erreichen, dass dessen Schwenkebene eine größere tangentiale Komponente aufweist und dass das betreffende Schalenteil in Umfangsrichtung weniger ausladend bauen kann.

Nach einer Ausführungsform der Erfindung wird vorgeschlagen, dass in einer Wandung des Schalenteils ein vorzugsweise durch die Wandung hindurchgehender Schlitz mit einer Schlitzebene vorgesehen ist, und dass in diesem Schlitz das Schwenkhebelelement schwenkbar angeordnet ist, wobei eine Schwenkebene des Schwenkhebelelements mit der Schlitzebene koinzidiert. Die Schlitzebene verläuft vorzugsweise parallel zu der Längsrichtung jedoch nach radial außen versetzt zu der Längsrichtung.

Weiter erweist es sich als vorteilhaft, wenn das Schwenkhebelelement eine Lagerachse aufweist, die von einem Stift gebildet ist, der in einem Schlitz in der Wandung des Schalenteils angeordnet ist, und dass der Stift orthogonal zu einer Ebene des Schlitzes angeordnet ist.

Die Verlängerungsvorrichtung ist weiter vorzugsweise so ausgebildet, dass beim Fügen des Kopfs des Knochenankers mit dem Schalenteil der Kopf und das Schalenteil ungefähr in Längsrichtung gegeneinander bewegbar sind und dass der Kopf des Knochenankers dabei gegen die erste Kontaktzone des Schwenkhebelelements anläuft und das Schwenkhebelelement im Bereich dieser ersten Kontaktzone, insbesondere nach außen, verdrängt, wodurch eine Stellbewegung bei der zweiten Kontaktzone in der Längsrichtung nach distal bewirkbar ist.

Wenn in der Folge der Kopf des Knochenankers gegen die zweite Kontaktzone anläuft, so kann er gleichwohl aufgrund der federelastischen Ausbildung oder des federelastischen Abschnitts des Schwenkhebelelements noch etwas weiter bewegt werden, so dass der Vorsprung des Schalenteils in den hintergreifbaren Bereich am Kopf des Knochenankers einrasten bzw. eingreifen kann. Aufgrund der federelastischen Stellkräfte ist dieser Hintergriff dann in Längsrichtung stabilisiert, so dass das betreffende Schalenteil stabil und beim bestimmungsgemäßen Gebrauch starr am Knochenanker gehalten ist.

Weiter kann vorgesehen sein, dass das Schwenkhebelelement, wenn es im Bereich der ersten Kontaktzone verdrängt wird, gegen einen die Verdrängung begrenzenden Anschlag anläuft. Dieser Anschlag ist bei einer beispielhaften Ausführungsform in einem das Schwenkhebelelement aufnehmenden Schlitz in der Wandung des Schalenteils ausgebildet.

Weiter kann es sich als vorteilhaft erweisen, wenn das Schwenkhebelelement mit seiner Schwenkebene parallel zur Längsachse jedoch beabstandet zur Längsachse angeordnet ist, so dass das Schwenkhebelelement mit seiner ersten Kontaktzone seitlich gegen einen Schenkel eines in einer Seitenansicht U-förmigen Kopfs des Knochenankers anliegt. Auf diese Weise kann das Schwenkhebelelement das Schalenteil zusätzlich in der Umfangsrichtung drehfest stützen.

Weiter erweist es sich als vorteilhaft, wenn ein zweites Schwenkhebelelement mit seiner Schwenkebene parallel zur Längsachse jedoch beabstandet zur Längsachse angeordnet ist, so dass das Schwenkhebelelement mit seiner ersten Kontaktzone seitlich gegen den Schenkel des Kopfs des Knochenankers auf einer in Umfangsrichtung dem ersten Schwenkhebelelement gegenüberliegenden Seite anliegt. Der betreffende Schenkel des U-förmigen Kopfs des Knochenankers vermag dann mit seinen beiden Längsflanken gegen die jeweilige erste Kontaktzone der beiden Schwenkhebelelemente anzulaufen. Hierdurch lässt sich eine gleichmäßige Krafteinleitung bei der Ausbildung der Montageverbindung des Schalenteils erreichen.

Es erweist sich weiter als vorteilhaft, wenn das Schalenteil einen nach innen eingezogenen Längsrandbereich aufweist, mit dem es von in Umfangsrichtung gegenüberliegenden Seiten gegen einen Schenkel eines in der Seitenansicht U-förmigen Kopfs des Knochenankers anlegbar ist, so dass das Schalenteil hierdurch in der Umfangsrichtung unverdrehbar am Kopf des Knochenankers anordenbar ist. Durch diese Maßnahme kann ungeachtet der Kraftausübung durch das Schwenkhebelelement eine in Umfangsrichtung drehfeste Anordnung des Schalenteils am Kopf des Knochenankers erzielt werden.

Des Weiteren erweist es sich als ganz besonders vorteilhaft, wenn das Schalenteil eine erste Abstützkontaktfläche und eine zweite Abstützkontaktfläche aufweist und dass die erste Abstützkontaktfläche distal zu dem radialen Vorsprung zum Hintergreifen des hintergreifbaren Bereichs am Kopf des Knochenankers vorgesehen ist und dass die zweite Abstützkontaktfläche proximal zu dem radialen Vorsprung zum Hintergreifen des hintergreifbaren Bereichs am Kopf des Knochenankers vorgesehen ist, so dass das Schalenteil distal und proximal zu dem Vorsprung flächenhaft gegen eine Außenseite des Kopfs des Knochenankers anlegbar und abstützbar ist. Auf diese Weise lässt sich eine besonders vorteilhafte Stabilisierung des betreffenden Schalenteils gegen die Außenseite des Kopfs des Knochenankers erzielen. Da die Außenseite des Kopfs des Knochenankers und dementsprechend die erste und die zweite Abstützkontaktfläche des Schalenteils gekrümmt, insbesondere bogenförmig oder zylindrisch gekrümmt, ausgebildet sind, lässt sich ein Verkippen des Schalenteils relativ zum Kopf des Knochenankers vollständig vermeiden. Das Schalenteil ist dann bezüglich aller sechs Freiheitsgrade stabilisiert und unbewegbar am Kopf des Knochenankers gehalten, jedenfalls so lange wie die durch das Schwenkhebelelement ausgeübten Stellkräfte nicht bewusst und intendiert überwunden werden, um das Schalenteil wieder zu lösen.

Weiter erweist es sich als vorteilhaft, wenn der radiale Vorsprung des jeweiligen Schalenteils in der Umfangsrichtung erstreckt ist und eine Neigung, Anschrägung oder Erstreckung in proximaler Richtung aufweist, so dass er mit einem ungefähr komplementär ausgebildeten und in der Umfangsrichtung erstreckten hintergreifbaren Bereich am Kopf des Knochenankers in der Längsrichtung und in der radialen Richtung einen Hintergriff ausbilden kann.

Gegenstand der Erfindung ist auch eine Sachgesamtheit aus einer Verlängerungsvorrichtung nach einem oder mehreren der vorstehenden Ansprüche und einem Knochenanker, insbesondere einer Pedikelschraube für die Wirbelsäulenchirurgie. Hierbei erweist sich als vorteilhaft, wenn der Knochenanker, insbesondere die Pedikelschraube, zwei in der Längsrichtung erstreckte Schenkel mit Innengewinde und je einer Sollbruchstelle aufweist, um die Schenkel nach der Implantation an der Sollbruchstelle abknicken und hierdurch kürzen zu können. Wenn ein solcher Knochenanker mit "Langkopf" verwendet wird, so lässt sich dessen verlängerter Innengewindebereich zum Einschrauben und Fixieren von weiteren Handhabungseinrichtungen und Instrumenten während der Implantation nutzen.

Weitere Merkmale, Einzelheiten und Vorteile der erfindungsgemäßen Verlängerungsvorrichtung ergeben sich aus den beigefügten Patentansprüchen und aus der zeichnerischen Darstellung und nachfolgenden Beschreibung erfindungsgemäßer Ausführungsformen der Erfindung.

In den Zeichnungen zeigen:
- Figur 1a: eine perspektivische Ansicht eines Knochenankers in Form einer Pedikelschraube und einer erfindungsgemäßen Verlängerungsvorrichtung mit zwei Schalenteilen;
- Figur 1b: die Komponenten der Figur 1a in einer lösbaren Halteverbindung;
- Figur 2a: eine perspektivische Ansicht eines Schalenteils mit zwei Schwenkhebelelementen und Lagerachsen;
- Figur 2b: die Komponenten nach Figur 2a in einem Montagezustand;
- Figuren 2c, d: verschiedene Ansichten des Schalenteils nach Figur 2b mit verschiedenen Betrachtungsebenen;
- Figuren 3a, b: verschiedene Ansichten des Schwenkhebelelements nach Figur 2a;
- Figuren 4a bis d: zwei Seitenansichten und zwei Schnittansichten von Knochenanker und Schalenteilen gemäß Figur 1b;
- Figuren 5a, b: perspektivische Ansichten eines Schalenteils mit einem Schwenkhebelelement nach einer weiteren Ausführungsform der erfindungsgemäßen Verlängerungsvorrichtung;
- Figuren 6a, b: weitere Ansichten der Verlängerungsvorrichtung nach Figuren 5b mit verschiedenen Betrachtungsebenen;
- Figuren 7a, b: Ansichten des Schwenkhebelelements gemäß Figur 5a.

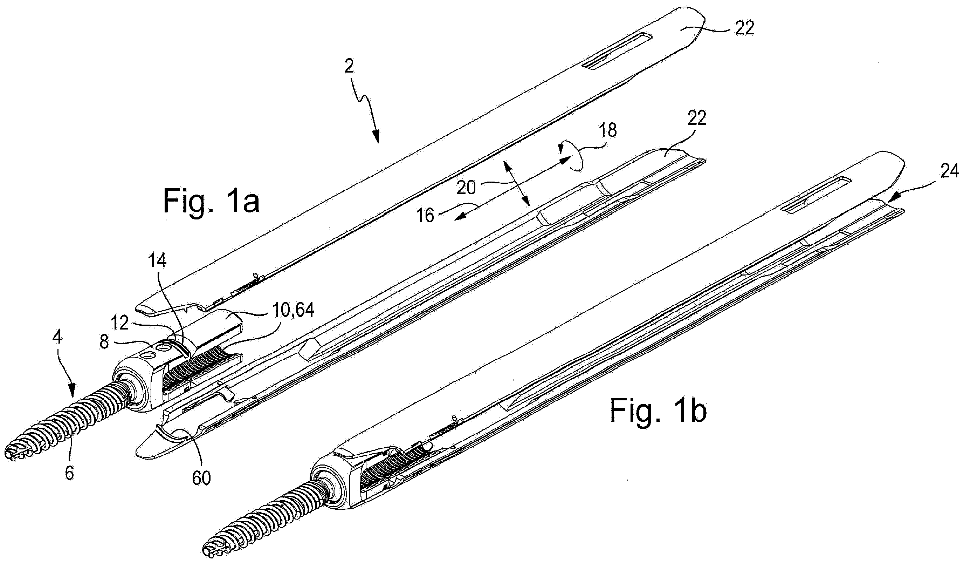

Die Figuren 1 bis 4 zeigen eine erste erfindungsgemäße Ausführungsform der erfindungsgemäßen Verlängerungsvorrichtung 2, teilweise in einer lösbaren Halteverbindung an einem Knochenanker 4 in Form einer beispielhaft dargestellten Pedikelschraube. Der Knochenanker 4 umfasst einen Schraubenschaft 6, der polyaxial verschwenkbar bezüglich eines bei einer seitlichen Betrachtung U-förmigen Kopfs 8 der Pedikelschraube 4 angeordnet ist. Bei dem Kopf 8 handelt es sich beispielhaft um einen sogenannten "Langkopf", der mit längeren Schenkeln 10 ausgebildet ist, wobei eine Sollbruchstelle 12 vorgesehen ist, an der der "Langkopf" im implantierten Zustand gekürzt werden kann. Dessen ungeachtet umfasst der Kopf 8 des Knochenankers 4 in an sich bekannter Weise einen hintergreifbaren Bereich 14, der in Form einer hintergreifbaren Nut am Außenumfang des Kopfs 8 ausgebildet ist. Der hintergreifbare Bereich 14 des Kopfs 8 kann im Übrigen entsprechend der Offenbarung in der Patentanmeldung DE 10 2014 225 327.6 der Anmelderin ausgebildet sein, deren Inhalt in die Offenbarung der vorliegenden Anmeldung einbezogen werden soll.

Die Verlängerungsvorrichtung 2 umfasst eine axiale Längsrichtung 16, eine hierzu konzentrische Umfangsrichtung 18 und eine radiale Richtung 20, die sich gleichermaßen auf den Kopf 8 des Knochenankers 4 beziehen. Die Verlängerungsvorrichtung 2 umfasst zwei voneinander separate und in der Längsrichtung 16 erstreckte Schalenteile 22, die beispielhaft und vorzugsweise einander entsprechend ausgebildet sind. Die Schalenteile 22 sind im Wesentlichen starr und verwindungsstabil ausgebildet in dem Sinne, dass sie nahezu vollständig formstabil sind, was aber eine geringfügige unbeachtliche Verformbarkeit insbesondere aufgrund der Länge nicht ausschließt. Die Schalenteile 22 sind auf noch zu beschreibende Weise lösbar an dem Kopf 8 des Knochenankers 4 befestigbar und bilden dann einen zwischen ihnen begrenzten Arbeitskanal 24, durch den der Chirurg mit weiteren Instrumenten Zugriff auf den Knochenanker 4 und das Operationsumfeld nehmen kann.

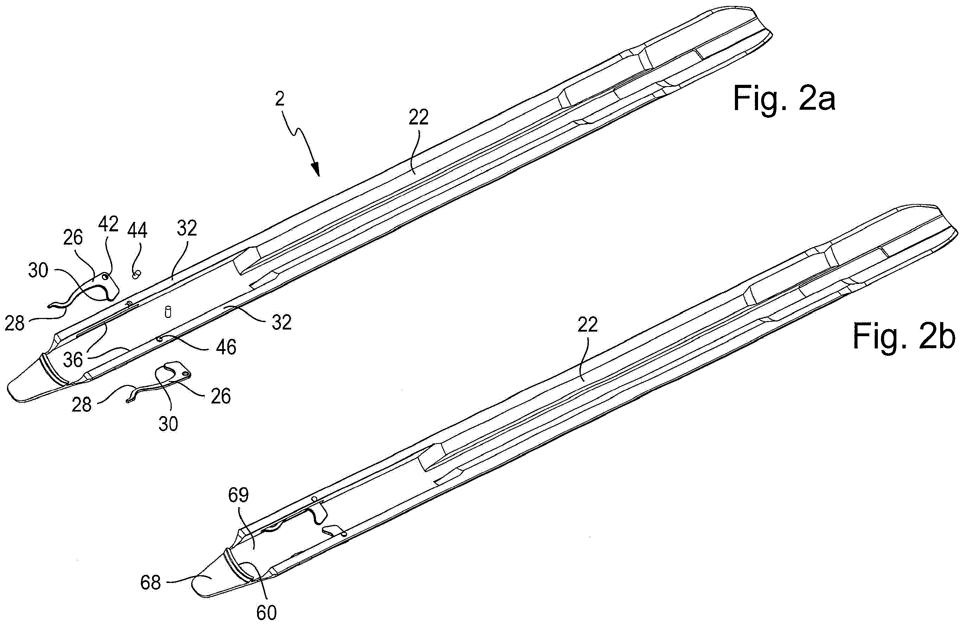

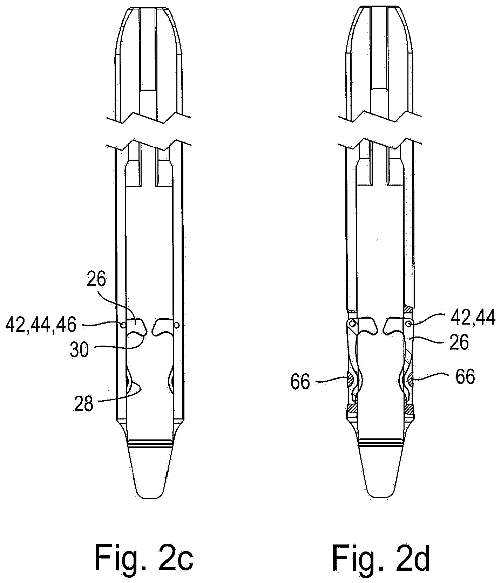

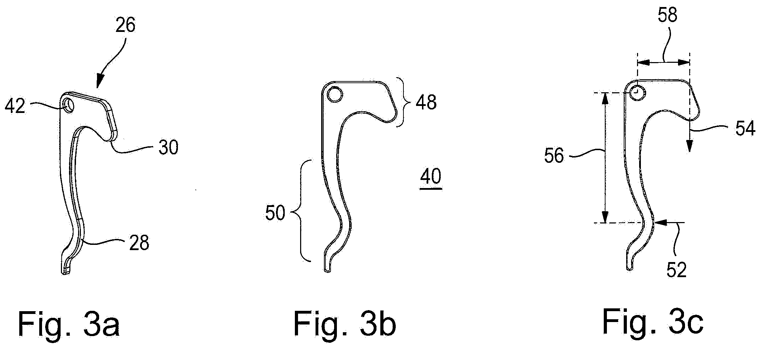

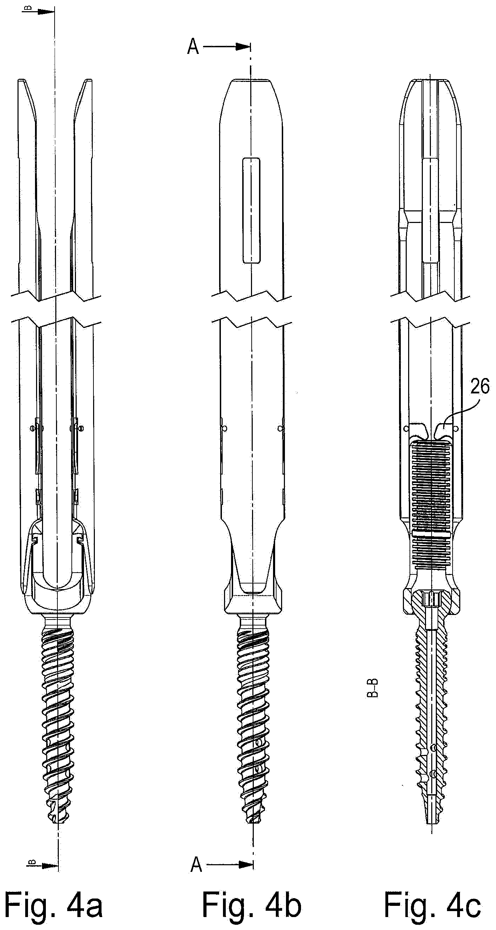

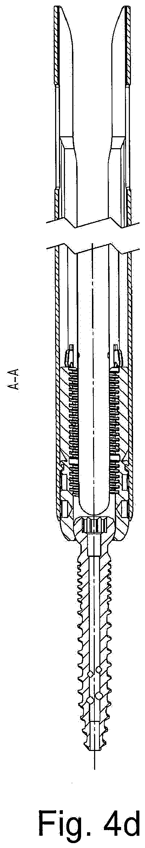

Wie am besten aus Figuren 2a, b ersehen werden kann, weist die Verlängerungsvorrichtung 2 bzw. ein jeweiliges Schalenteil 22 jeweils vorzugsweise zwei Schwenkhebelelemente 26 auf, die im Einzelnen in Figuren 3a, b dargestellt sind. Das jeweilige Schwenkhebelelement 26 umfasst eine erste Kontaktzone 28 und eine zweite Kontaktzone 30. Die erste Kontaktzone 28 ist quer zur Längsrichtung 16 gegen den Kopf 8 des Knochenankers 4 anlegbar. Die zweite Kontaktzone 30 ist in der Längsrichtung 19 in distaler Richtung gegen ein proximales Ende des Kopfs 8 anlegbar (vgl. Figur 4c). Hierbei ist das jeweilige Schwenkhebelelement 26 auf noch näher zu beschreibende Weise in nach innen eingezogenen Längsrandbereichen 32 des Schalenteils 22 beweglich, jedoch verliersicher in der Wandung 34 aufgenommen. Hierfür ist in der Wandung 34 des Schalenteils 22, und zwar in dem jeweiligen nach innen eingezogenen Längsrandbereich 32, ein durchgehender Schlitz 36 ausgebildet, in dem das jeweilige Schwenkhebelelement 26 schwenkbar aufgenommen ist. Der Schlitz 36 weist daher eine Schlitzebene 38 auf, die mit einer Schwenkebene 40 des Schwenkhebelelements 26 koinzidiert (Zeichnungsebene der Figur 3b). Zur verliersicheren Lagerung des Schwenkhebels 26 in dem Schlitz 36 umfasst das Schwenkhebelelement 26 eine Lageröffnung 42, durch die eine stiftförmige Lagerachse 44 hindurchgreift. Auch der Längsrandbereich 32 umfasst eine sacklochförmige Öffnung 46, durch welche die Lagerachse 44 eingefügt werden kann. Bei additiven Fertigungsverfahren wäre aber auch eine einstückige Ausbildung der Lagerachse 44 mit dem Schalenteil 22 denkbar. Der jeweilige Schwenkhebel 26 könnte insgesamt federelastisch ausgebildet sein (nicht dargestellt), im vorliegenden Fall umfasst er einen im Wesentlichen steifen oder starren Abschnitt 48 und einen federelastischen Abschnitt 50 (vgl. Figur 3b), die vorliegend eher kontinuierlich ineinander übergehen. Wenn mittels des Kopfs 8 des Knochenankers 4 eine verdrängende Kraft in Richtung des Pfeils 52, also im Wesentlichen quer zur Längsrichtung 16, auf die erste Kontaktzone 28 ausgeübt wird, so wird das Schwenkhebelelement 26 um seine Lagerung verschwenkt, wodurch die zweite Kontaktzone 30 in Richtung des Pfeils 54 verschwenkt wird. Trotz einer gewissen Kraftabsorption durch die federelastische Verformung sind ein erster längerer Hebelarm 56 und ein zweiter kürzerer Hebelarm 58 gegeben.

Zum lösbaren Verbinden des Schalenteils 22 mit dem Kopf 8 des Knochenankers 4 umfasst das jeweilige Schalenteil 22 einen in der Umfangsrichtung 20 erstreckten radialen Vorsprung 60, der komplementär zu dem hintergreifbaren Bereich 14 am Kopf 8 des Knochenankers 4 ausgebildet ist. Der radiale Vorsprung 60 umfasst zudem eine Neigung, Anschrägung oder Erstreckung in proximaler Längsrichtung 16, so dass er in den hintergreifbaren Bereich 14 eingreifen kann. Zum Fügen des jeweiligen Schalenteils 22 an den Kopf 8 des Knochenankers 4 wird das Schalenteil zunächst von radial außen und aus proximaler Richtung an die Außenseite des Kopfs 8 angelegt, derart, dass ein proximales Ende 62 des Kopfs 8 des Knochenankers bezüglich des Vorsprungs 60 noch distal positioniert ist. Sodann wird das Schalenteil 22 in distaler Längsrichtung 16 in Richtung auf den betreffenden Schenkel 10 des Kopfs 8 aufgeschoben. Dabei läuft der Schenkel 10 mit seinen beiderseitigen Flanken 64 gegen die jeweilige erste Kontaktzone 28 des jeweiligen Schwenkhebelelements 26 an und verdrängt diese nach außen. In der Folge verschwenkt die zweite Kontaktzone 30, und der Schenkel 10 des Kopfs 8 läuft mit seinem proximalen Ende 62 in der Längsrichtung 16 gegen die zweite Kontaktzone 30 des Schwenkhebelelements 26 an. In dieser Position ist der radiale Vorsprung 60 des Schalenteils noch außer Eingriff mit dem hintergreifbaren Bereich 14 des Kopfs 8 des Knochenankers. Wenn jetzt aber das Schalenteil 22 geringfügig weiter in distaler Längsrichtung aufgeschoben wird, so kommt es zu einer weiteren elastischen Verformung innerhalb des Schwenkhebelelements 26, welche das Eingreifen oder Einrasten des radialen Vorsprungs 60 in den hintergreifbaren Bereich 14 am Kopf des Knochenankers gestattet, was dann auch mit einer geringfügigsten proximalen Rückstellbewegung des Schalenteils 22 einhergeht. Im Ergebnis ist dann das betreffende Schalenteil in allen sechs Freiheitsgraden starr am Kopf 8 des Knochenankers 4 stabil gehalten und durch die über das Schwenkhebelelement 26 ausgeübten Kräfte stabilisiert. Es sei auch darauf hingewiesen, dass für die stabile Anordnung eine erste und eine zweite Abstützkontaktfläche 66, 48 bei den Schalenteilen 22 sehr hilfreich ist, die sich distal bzw. proximal zu dem radialen Vorsprung 60 befinden und entsprechend wie die Außenseite der Schenkel 10 des Kopfs 8 des Knochenankers 4 gewölbt sind, so dass das betreffende Schalenteil 22 flächenhaft gegen den Kopf 8 anliegen kann. Ein Verkippen des Schalenteils 22 bezüglich des Kopfs 8 ist dann nicht mehr möglich.

Man erkennt insbesondere aus Figur 2d, dass im Bereich der ersten Kontaktzone 28 des Schwenkhebelelements ein die Verdrängung begrenzender Anschlag 66 vorgesehen ist, der vorliegend beispielhaft als ein die Schlitzbreite überbrückender Steg ausgebildet ist. Hierdurch wird verhindert, dass das Schwenkhebelelement 26 nach radial außen über den Außenumfang des Schalenteils 22 nach außen in das Operationsfeld eintritt.

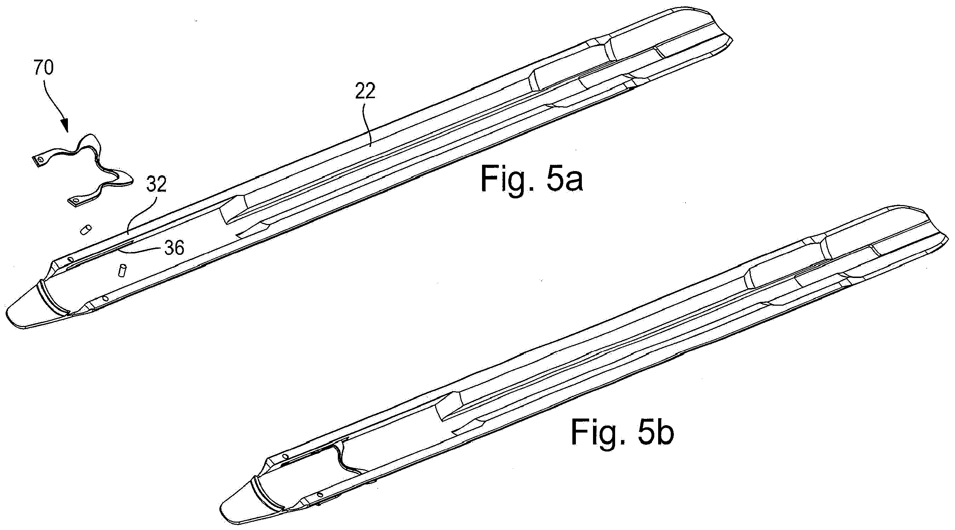

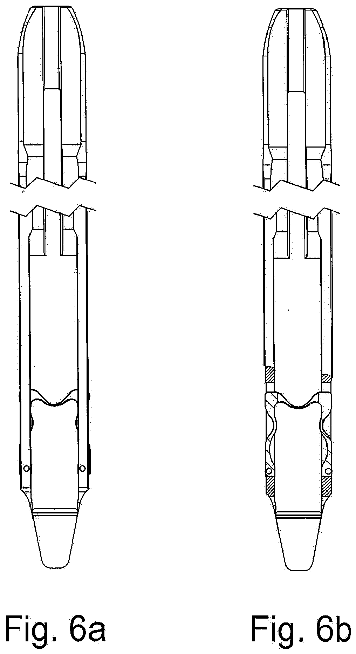

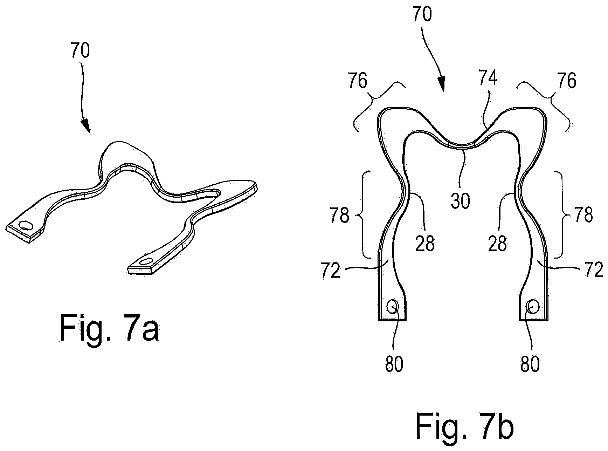

Die Figuren 5 bis 7 zeigen eine Verlängerungsvorrichtung mit einem U-förmigen Schwenkhebelelement 70 mit zwei Schenkeln 72 und einem diese zwei Schenkel 70 verbindenden Querschenkel 74. Man erkennt wiederum eine erste Kontaktzone 28 bei dem jeweiligen Schenkel 72 und eine zweite Kontaktzone 30 bei dem Querschenkel 74. Bei diesem Ausführungsbeispiel umfasst das Schwenkhebelelement 70 jeweils im Übergangsbereich des Querschenkels 74 zu den beiden Schenkeln 72 einen im Wesentlichen starren Abschnitt 76. Daran anschließend ist ein federelastischer Abschnitt 78 ausgebildet. In Richtung auf die distalen Enden der Schenkel 72 ist das Schwenkhebelelement 70 wieder eher starr ausgebildet. Dort sind dann auch Lageröffnungen 80 zur schwenkbaren Anordnung und Lagerung des Schwenkhebelelements 70 in einem Schlitz 36 wiederum in einem nach innen eingezogenen Längsrandbereich 32 des Schalenteils 22 ausgebildet. Wie man aus Figuren 7a, b erkennt, ist das Schwenkhebelelement 70 nicht als durchgehend ebenes in einer einzigen Ebene erstrecktes Bauteil ausgebildet, sondern sein Querschenkel 74 ist gekrümmt, so dass sich das insgesamt U-förmige Schwenkhebelelement 70 einer gewissen Umfangserstreckung des Schalenteils 22 anzupassen vermag. Das U-förmige Schwenkhebelelement 70 stellt gewissermaßen eine Kombination aus zwei L-förmigen Schwenkhebelelementen 26 der ersten Ausführungsform dar. Die grundsätzliche Funktionsweise ist jedoch dieselbe. Es wäre auch insbesondere denkbar, dass die Lageröffnungen 80 nicht wie dargestellt am distalen Ende der Schenkel 72, sondern im Bereich des starren Abschnitts 76, also eher vergleichbar zu dem ersten Ausführungsbeispiel, angeordnet sind.

## Patentansprüche

1. Verlängerungsvorrichtung (2) für einen Knochenanker (4), insbesondere für eine Knochenschraube, insbesondere für eine Pedikelschraube, insbesondere in der minimalinvasiven Wirbelsäulenchirurgie, mit einer axialen Längsrichtung (16), einer hierzu konzentrischen Umfangsrichtung (18) und einer radialen Richtung (20), wobei die Verlängerungsvorrichtung (2) in der axialen Längsrichtung (16) erstreckt ist und mittels eines radialen Vorsprungs (60) einen hintergreifbaren Bereich (14) am Kopf (8) des Knochenankers (4) in der Längsrichtung (16) und zudem in radialer Richtung 20 hintergreift und in entgegengesetzter Richtung gegen den Kopf (8) des Knochenankers (4) abstützbar ist, so dass die Verlängerungsvorrichtung hierdurch am Kopf (8) des Knochenankers lösbar, jedoch starr und zudem drehfest fixierbar ist, wobei die Verlängerungsvorrichtung wenigstens ein und vorzugsweise zwei voneinander separate, und in der Längsrichtung (16) erstreckte im wesentlichen starre Schalenteile (22) umfasst, von denen jedes einen radialen Vorsprung (60) zum Hintergreifen des hintergreifbaren Bereichs (14) am Kopf (8) des Knochenankers (4) umfasst,
wobei an wenigstens einem Schalenteil (22) und vorzugsweise an beiden Schalenteilen (22) wenigstens ein Schwenkhebelelement (26, 70) vorgesehen ist,
wobei das Schwenkhebelelement (26, 70) eine erste und eine zweite Kontaktzone (28, 30) zu dem Kopf (8) des Knochenankers (4) aufweist,
wobei die erste Kontaktzone (28) des Schwenkhebelelements (26, 70) quer zur Längsrichtung (16) gegen den Kopf (8) des Knochenankers anlegbar ist und wobei die zweite Kontaktzone (30) des Schwenkhebelelements (26, 70) in der Längsrichtung (16) in distaler Richtung gegen den Kopf (8) des Knochenankers anlegbar ist,
wobei das Schwenkhebelelement (26, 70) federelastisch ausgebildet ist oder wenigstens einen im wesentlichen starren Abschnitt (48, 76) und wenigstens einen federelastischen Abschnitt (50, 78) aufweist derart, dass eine Auslenkung des Schwenkhebelelements (26, 70) über die erste Kontaktzone (28) durch den Kopf (8) des Knochenankers zu einer Kraftbeaufschlagung und gegebenenfalls zusätzlich zu einer Auslenkung des Schwenkhebelelements (26, 70) im Bereich der zweiten Kontaktzone (30) auf den Knochenanker führt, so dass in der Folge das Schalenteil (22) mittels seines Schwenkhebelelements (26, 70) in der Längsrichtung (16) unverschieblich am Kopf (8) des Knochenankers angeordnet ist, wenn sein radialer Vorsprung (60) in Eingriff mit dem hintergreifbaren Bereich (14) am Kopf (8) des Knochenankers ist.

2. Verlängerungsvorrichtung nach Anspruch 1, **dadurch gekennzeichnet, dass** das Schwenkhebelelement mit seiner zweiten Kontaktzone gegen ein proximales Ende des Kopfs des Knochenankers anlegbar ist.

3. Verlängerungsvorrichtung nach einem oder mehreren der vorstehenden Ansprüche, **dadurch gekennzeichnet, dass** das Schwenkhebelelement (26) in einer Ebene erstreckt ist, die insbesondere parallel zur Längsrichtung (16) erstreckt ist.

4. Verlängerungsvorrichtung nach einem oder mehreren der vorstehenden Ansprüche, **dadurch gekennzeichnet, dass** das Schwenkhebelelement (26, 70) eine Schwenk- oder Lagerachse (44) aufweist, die orthogonal zu der Längsrichtung (16) jedoch nach radial außen versetzt zu dieser verläuft.

5. Verlängerungsvorrichtung nach einem oder mehreren der vorstehenden Ansprüche, **dadurch gekennzeichnet, dass** in einer Wandung (34) des Schalenteils (22) ein vorzugsweise durch die Wandung (34) hindurchgehender Schlitz (36) mit einer Schlitzebene (38) vorgesehen ist, und dass in diesem Schlitz (36) das Schwenkhebelelement (26, 70) schwenkbar angeordnet ist, wobei eine Schwenkebene (40) des Schwenkhebelelements (26, 70) mit der Schlitzebene (38) koinzidiert.

6. Verlängerungsvorrichtung nach einem oder mehreren der vorstehenden Ansprüche, **dadurch gekennzeichnet, dass** das Schwenkhebelelement (26, 70) eine Lagerachse (44) aufweist, die von einem Stift gebildet ist, der in einem Schlitz (36) in der Wandung (34) des Schalenteils (22, 70) angeordnet ist, und dass der Stift orthogonal zu einer Ebene (38) des Schlitzes angeordnet ist.

7. Verlängerungsvorrichtung nach einem oder mehreren der vorstehenden Ansprüche, **dadurch gekennzeichnet, dass** beim Fügen des Kopfs (8) des Knochenankers (4) mit dem Schalenteil (22, 70) der Kopf (8) und das Schalenteil (22, 70) ungefähr in Längsrichtung (16) gegeneinander bewegbar sind und dass der Kopf (8) des Knochenankers dabei gegen die erste Kontaktzone (28) des Schwenkhebelelements (26) anläuft und das Schwenkhebelelement (26) im Bereich dieser ersten Kontaktzone (28), insbesondere nach außen, verdrängt, wodurch eine Stellbewegung bei der zweiten Kontaktzone (30) in der Längsrichtung (16) nach distal bewirkbar ist.

8. Verlängerungsvorrichtung nach einem oder mehreren der vorstehenden Ansprüche, **dadurch gekennzeichnet, dass** das Schwenkhebelelement (26) wenn es im Bereich der ersten Kontaktzone (28) verdrängt wird, gegen einen die Verdrängung begrenzenden Anschlag (66) anläuft.

9. Verlängerungsvorrichtung nach Anspruch 8, **dadurch gekennzeichnet, dass** dieser Anschlag (66) in einem das Schwenkhebelelement (26) aufnehmenden Schlitz (36) in der Wandung (34) des Schalenteils (22) ausgebildet ist.

10. Verlängerungsvorrichtung nach einem oder mehreren der vorstehenden Ansprüche, **dadurch gekennzeichnet, dass** das Schwenkhebelelement (26) mit seiner Schwenkebene (40) parallel zur Längsachse (16) jedoch beabstandet zur Längsachse (16) angeordnet ist, so dass das Schwenkhebelelement (26) mit seiner ersten Kontaktzone (28) seitlich gegen einen Schenkel (10) eines in einer Seitenansicht U-förmigen Kopfs (8) des Knochenankers (4) anliegt.

11. Verlängerungsvorrichtung nach Anspruch 10, **dadurch gekennzeichnet, dass** ein zweites Schwenkhebelelement (26) mit seiner Schwenkebene (40) parallel zur Längsachse (16) jedoch beabstandet zur Längsachse angeordnet ist, so dass das Schwenkhebelelement (26) mit seiner ersten Kontaktzone (28) seitlich gegen den Schenkel (10) des Kopfs (8) des Knochenankers auf einer in Umfangsrichtung (18) dem ersten Schwenkhebelelement (26) gegenüberliegenden Seite anliegt.

12. Verlängerungsvorrichtung nach einem oder mehreren der vorstehenden Ansprüche, **dadurch gekennzeichnet, dass** das Schalenteil (22) einen nach innen eingezogenen Längsrandbereich (32) aufweist, mit dem es von in Umfangsrichtung (18) gegenüberliegenden Seiten gegen einen Schenkel (10) eines in der Seitenansicht U-förmigen Kopfs (8) des Knochenankers (4) anlegbar ist, so dass das Schalenteil (26) hierdurch in der Umfangsrichtung (18) unverdrehbar am Kopf (8) des Knochenankers (4) anordenbar ist.

13. Verlängerungsvorrichtung nach einem oder mehreren der vorstehenden Ansprüche, **dadurch gekennzeichnet, dass** das Schalenteil (26) eine erste Abstützkontaktfläche (68) und eine zweite Abstützkontaktfläche (69) aufweist und dass die erste Abstützkontaktfläche (68) distal zu dem radialen Vorsprung (60) zum Hintergreifen des hintergreifbaren Bereichs (14) am Kopf (8) des Knochenankers vorgesehen ist und dass die zweite Abstützkontaktfläche (69) proximal zu dem radialen Vorsprung (60) zum Hintergreifen des hintergreifbaren Bereichs am Kopf des Knochenankers vorgesehen ist, so dass das Schalenteil (26) distal und proximal zu dem Vorsprung (60) flächenhaft gegen eine Außenseite des Kopfs des Knochenankers anlegbar und abstützbar ist, wobei der radiale Vorsprung (60) des jeweiligen Schalenteils (26) vorzugsweise in der Umfangsrichtung (18) erstreckt ist und eine Neigung, Anschrägung oder Erstreckung in proximaler Richtung aufweist, so dass er mit einem ungefähr komplementär ausgebildeten und in der Umfangsrichtung erstreckten hintergreifbaren Bereich (14) am Kopf (8) des Knochenankers (4) in der Längsrichtung (16) und in der radialen Richtung (20) einen Hintergriff ausbilden kann.

14. Sachgesamtheit aus einer Verlängerungsvorrichtung (2) nach einem oder mehreren der vorstehenden Ansprüche und einem Knochenanker (4), insbesondere einer Pedikelschraube, für die Wirbelsäulenchirurgie.

15. Sachgesamtheit nach Anspruch 14, **dadurch gekennzeichnet, dass** der Knochenanker (4), insbesondere die Pedikelschraube, zwei in der Längsrichtung (16) erstreckte Schenkel (26) mit Innengewinde und je einer Sollbruchstelle (12) aufweist, um die Schenkel nach der Implantation an der Sollbruchstelle (12) abknicken und hierdurch kürzen zu können.
